# EUROPEAN PATENT APPLICATION

(11) **EP 0 529 622 A2**
(43) Date of publication of application: **03.03.1993**
(21) Application number: 92114637.9
(22) Date of filing: 27.08.1992
(51) Int. Cl.: C12N 15/60, C12N 9/88, C12N 1/21, C12P 21/08, C12Q 1/68, A61K 37/56

(54) **Cloning and characterization of a cardiac adenylyl cyclase**

(30) Priority: 29.08.1991 US 751460
(71) Applicant: AMERICAN CYANAMID COMPANY, Wayne, NJ 07470-8426 (US)
(72) Inventor: Ishikawa, Yoshihiro, Cresskill, New Jersey (US)
(74) Representative: Wächtershäuser, Günter, Prof. Dr.

(57) **Abstract**

A DNA sequence encoding a novel effector enzyme referred to as a cardiac adenylyl cyclase is described. The amino acid sequence of the cardiac adenylyl cyclase encoded by that DNA sequence is also described.

SEQUENCE LISTING

(1) GENERAL INFORMATION:

(i) APPLICANT: Yoshihiro Ishikawa

(ii) TITLE OF INVENTION: Cloning and Character-
ization of a Cardiac Adenylyl Cyclase

(iii) NUMBER OF SEQUENCES: 1

(iv) CORRESPONDENCE ADDRESS:

(A) ADDRESSEE: Antoinette F. Konski
American Cyanamid Company
(B) STREET: 1937 West Main Street
P.O. Box 60

(C) CITY: Stamford

(D) STATE: Connecticut

(E) COUNTRY: USA

(F) ZIP: 06904

(v) COMPUTER READABLE FORM:

(A) MEDIUM TYPE: Floppy disk

(B) COMPUTER: IBM PC AT

(C) OPERATING SYSTEM: MS-DOS

(D) SOFTWARE:   ASCII from DW4

(vi) CURRENT APPLICATION DATA:

(A) APPLICATION NUMBER:

(B) FILING DATE:

(C) CLASSIFICATION:

(vii) PRIOR APPLICATION DATA:

(A) APPLICATION NUMBER:   751,460

(B) FILING DATE:   August 29, 1991

(viii) ATTORNEY/AGENT INFORMATION:

(A) NAME:   Antoinette F. Konski

(B) REGISTRATION NUMBER:   34,202

(C) REFERENCE/DOCKET NUMBER:  31,618-02

(ix) TELECOMMUNICATION INFORMATION:

(A) TELEPHONE:   203 321 2455

(B) TELEFAX:     203 321 2971

(C) TELEX:

(2) INFORMATION FOR SEQ ID NO: 1:

(i) SEQUENCE CHARACTERISTICS:

(A) LENGTH: 4356 base pairs

(B) TYPE: nucleic acid

(C) STRANDEDNESSS: single

(D) TOPOLOGY: linear

(ii) MOLECULE TYPE: cDNA

(xi) SEQUENCE DESCRIPTION: SEQ ID NO: 1:

CCAACGCGCA ACCCGCACGC CGCCGGGGGC CGACCCGCGG 40

CTGCAGCAAG AAGCCCGGGG GGGCGGTGAC CCCGCAGCTG 80

CAGCAGCAGC AGCAGCAGCA GCAGCACGAG CAGCAGCACG 120

AGCAGCAGCA GCACGAGCAG CAGCAGC 147

ATG TGC AGC AGC AGC AGC GCC TGG CCA AGC GCT GGC 183
Met Cys Ser Ser Ser Ser Ala Trp Pro Ser Ala Gly
1               5                   10

GCG GCG ACG ACG ACC CCC CGC TGG GCG GCG ACG ACC 219
Ala Ala Thr Thr Thr Pro Arg Trp Ala Ala Thr Thr
        15                  20

CCC TGG CCG GGG GCT TCG GCT TCA GCT TCC GCT CCA 255
Pro Trp Pro Gly Ala Ser Ala Ser Ala Ser Ala Pro
25                  30                  35

GGT CGG CCT GGC AGG AGC GCG GCG GCG ACG ACT GCG 291
Gly Arg Pro Gly Arg Ser Ala Ala Ala Thr Thr Ala
            40                  45

GGC GCG GCA GCC GGC GGC GGC GGC GGG GCG CGG CGG 327
Gly Ala Ala Ala Gly Gly Gly Gly Gly Ala Arg Arg
    50                  55                  60

GCG GGG GCA GCT CCC GGG CGC CCC TGC GGG CGG CGG 363
Ala Gly Ala Ala Pro Gly Arg Pro Cys Gly Arg Arg
                65                  70

CGG CGG CCC GGC GGC GGC GGG CGG GGC GGA GGT GCG 399
Arg Arg Pro Gly Gly Gly Gly Arg Gly Gly Gly Ala
        75                  80

CCC CCG CTC GGT GGA GCT GGG CCT GGA CGA GCG GCG 435
Pro Pro Leu Gly Gly Ala Gly Pro Gly Arg Ala Ala
85                  90                  95

GGG CCG GGG CCG CGC CGA GCC CGA GCC CGA GGC CGA 471
Gly Pro Gly Pro Arg Arg Ala Arg Ala Arg Gly Arg
            100                 105

GGC CGG CGC CCC CGG GGG CGA CCG CGG GGC GCG GGA 507
Gly Arg Arg Pro Arg Gly Arg Pro Arg Gly Ala Gly
    110                 115                 120

CGG CGA CGG CCC GCA GGG CCG GCG GCC TGC TGC CGC 543
Arg Arg Arg Pro Ala Gly Pro Ala Ala Cys Cys Arg
                125                 130

GCG CTG CTG CAG ATC TTC CGC TCC AAG AAG TTC CCG 579
Ala Leu Leu Gln Ile Phe Arg Ser Lys Lys Phe Pro
        135                 140

TCG GAC AAG CTG GAG CGG CTG TAC CAG CGC TAC TTC 615
Ser Asp Lys Leu Glu Arg Leu Tyr Gln Arg Tyr Phe
145                 150                 155

TTC CGC CTG AAC CAG AGC AGC CTC ACC ATG CTC ATG 651
Phe Arg Leu Asn Gln Ser Ser Leu Thr Met Leu Met
            160                 165

GCC GTG CTG GTG CTC GTG TGC CTC GTG ATG CTG GCC 687
Ala Val Leu Val Leu Val Cys Leu Val Met Leu Ala
    170                 175                 180

TTC CAC GCG GCG CGG CCC CCG CTG CGG CTG CCC CAC 723
Phe His Ala Ala Arg Pro Pro Leu Arg Leu Pro His
                185                 190

CTG GCC GTG CTG GCG GCC GCG GTC GGG GTC ATC CTG 759
Leu Ala Val Leu Ala Ala Ala Val Gly Val Ile Leu
        195                 200

GTC ATG GCC GTG CTC TGC AAC CGC GCC GCC TTC CAC 795
Val Met Ala Val Leu Cys Asn Arg Ala Ala Phe His
205                 210                 215

CAG GAC CAC ATG GGC CTG GCC TGC TAC GCG CTC ATC 831
Gln Asp His Met Gly Leu Ala Cys Tyr Ala Leu Ile
            220                 225

GCC GTG GTG CTG GCC GTG CAG GTG GTG GGC CTG CTG 867
Ala Val Val Leu Ala Val Gln Val Val Gly Leu Leu
    230                 235                 240

CTG CCC CAG CCG CGC AGC GCC TCC GAG GGC ATC TGG 903
Leu Pro Gln Pro Arg Ser Ala Ser Glu Gly Ile Trp
                245                 250

TGG ACC GTG TTC TTC ATC TAC ACC ATC TAC ACG CTG 939
Trp Thr Val Phe Phe Ile Tyr Thr Ile Tyr Thr Leu
        255                 260

CTG CCC GTG CGC ATG CGG GCC GCC GTC CTC AGC GGA 975
Leu Pro Val Arg Met Arg Ala Ala Val Leu Ser Gly
265                 270                 275

GTG CTC CTG TCG GCC CTG CAC CTG GCC ATC GCC CTG 1011
Val Leu Leu Ser Ala Leu His Leu Ala Ile Ala Leu
            280                 285

CGC GCC AAC GCC CAG GAC CGG TTC CTG CTC AAG CAG 1047
Arg Ala Asn Ala Gln Asp Arg Phe Leu Leu Lys Gln
    290                 295                 300

CTC GTC TCC AAT GTC CTC ATT TTC TCC TGC ACC AAC 1083
Leu Val Ser Asn Val Leu Ile Phe Ser Cys Thr Asn
                305                 310

ATC GTG GGT GTC TGT ACC CAC TAC CCG GCT GAG GTC 1119
Ile Val Gly Val Cys Thr His Tyr Pro Ala Glu Val
        315                 320

TCC CAG AGA CAG GCC TTC CAA GAG ACC CGG GAG TGC 1155
Ser Gln Arg Gln Ala Phe Gln Glu Thr Arg Glu Cys
325                 330                 335

ATC CAG GCA CGG CTC CAC TCG CAA CGG GAG AAC CAG 1191
Ile Gln Ala Arg Leu His Ser Gln Arg Glu Asn Gln
            340                 345

CAA CAG GAG CGG CTC CTG CTG TCT GTC CTG CCC CGA 1227
Gln Gln Glu Arg Leu Leu Leu Ser Val Leu Pro Arg
    350                 355                 360

CAC GTT GCC ATG GAG ATG AAA GCA GAC ATC AAT GCC 1263
His Val Ala Met Glu Met Lys Ala Asp Ile Asn Ala
                365                 370

AAG CAG GAG GAT ATG ATG TTC CAT AAG ATT TAC ATC 1299
Lys Gln Glu Asp Met Met Phe His Lys Ile Tyr Ile
        375                 380

CAG AAA CAT GAC AAC GTG AGC ATC CTG TTT GCT GAC 1335
Gln Lys His Asp Asn Val Ser Ile Leu Phe Ala Asp
385                 390                 395

ATC GAG GGC TTC ACC AGC TTG GCA TCC CAG TGC ACT 1371
Ile Glu Gly Phe Thr Ser Leu Ala Ser Gln Cys Thr
            400                 405

GCC CAG GAG CTG GTC ATG ACG CTC AAT GAG CTC TTC 1407
Ala Gln Glu Leu Val Met Thr Leu Asn Glu Leu Phe
    410                 415                 420

GCC CGC TTC GAC AAG CTG GCT GCG GAG AAT CAC TGT 1443
Ala Arg Phe Asp Lys Leu Ala Ala Glu Asn His Cys
                425                 430

TTA CGT ATT AAG ATC CTG GGG GAT TGT TAT TAC TGT 1479
Leu Arg Ile Lys Ile Leu Gly Asp Cys Tyr Tyr Cys
        435                 440

GTC TCT GGG CTG CCT GAA GCG AGG GCC GAC CAC GCC 1515
Val Ser Gly Leu Pro Glu Ala Arg Ala Asp His Ala
445                 450                 455

CAC TGC TGC GTG GAG ATG GGC ATG GAC ATG ATT GAG 1551
His Cys Cys Val Glu Met Gly Met Asp Met Ile Glu
            460                 465

GCC ATC TCG TTG GTC CGG GAG GTG ACA GGG GTG AAC 1587
Ala Ile Ser Leu Val Arg Glu Val Thr Gly Val Asn
    470                 475                 480

GTG AAC ATG CGC GTG GGA ATT CAC AGC GGG CGA GTA 1623
Val Asn Met Arg Val Gly Ile His Ser Gly Arg Val
                485                 490

CAC TGC GGT GTC CTT GGT CTC AGG AAG TGG CAG TTC 1659
His Cys Gly Val Leu Gly Leu Arg Lys Trp Gln Phe
        495                 500

GAC GTC TGG TCT AAT GAC GTC ACG CTG GCC AAC CAT 1695
Asp Val Trp Ser Asn Asp Val Thr Leu Ala Asn His
505                 510                 515

ATG GAA GCT GGA GGC AAG GCT GGG CGC ATC CAC ATC 1731
Met Glu Ala Gly Gly Lys Ala Gly Arg Ile His Ile
            520                 525

ACC AAG GCC ACA CTC AGC TAC CTG AAC GGT GAC TAC 1767
Thr Lys Ala Thr Leu Ser Tyr Leu Asn Gly Asp Tyr
    530                 535                 540

GAG GTG GAG CCA GGC TGC GGG GGC GAG CGC AAC GCC 1803
Glu Val Glu Pro Gly Cys Gly Gly Glu Arg Asn Ala
                545                 550

TAC CTC AAG GAG CAC AGT ATC GAG ACC TTC CTC ATC 1839
Tyr Leu Lys Glu His Ser Ile Glu Thr Phe Leu Ile
        555                 560

CTG CGC TGC ACC CAG AAG CGG AAA GAA GAA AAG GCC 1875
Leu Arg Cys Thr Gln Lys Arg Lys Glu Glu Lys Ala
565                 570                 575

ATG ATC GCC AAG ATG AAT CGC CAG AGA ACC AAC TCC 1911
Met Ile Ala Lys Met Asn Arg Gln Arg Thr Asn Ser
            580                 585

ATT GGG CAC AAC CCG CCC CAC TGG GGG GCC GAA CGT 1947
Ile Gly His Asn Pro Pro His Trp Gly Ala Glu Arg
    590                 595                 600

CCC TTC TAC AAC CAC CTA GGA GGC AAC CAG GTG TCC 1983
Pro Phe Tyr Asn His Leu Gly Gly Asn Gln Val Ser
                605                 610

AAG GAG ATG AAG CGC ATG GGC TTC GAA GAC CCC AAG 2019
Lys Glu Met Lys Arg Met Gly Phe Glu Asp Pro Lys
        615                 620

GAC AAG AAC GCC CAG GAA AGT GCG AAC CCT GAG GAT 2055
Asp Lys Asn Ala Gln Glu Ser Ala Asn Pro Glu Asp
625                 630                 635

GAA GTG GAT GAA TTT CTG GGC CGC GCC ATT GAC GCC 2091
Glu Val Asp Glu Phe Leu Gly Arg Ala Ile Asp Ala
            640                 645

AGG AGC ATC GAC AGG CTG CGG TCG GAG CAC GTC CGC 2127
Arg Ser Ile Asp Arg Leu Arg Ser Glu His Val Arg
    650                 655                 660

AAG TTC CTC CTG ACC TTC AGG GAG CCT GAC TTA GAA 2163
Lys Phe Leu Leu Thr Phe Arg Glu Pro Asp Leu Glu
                665                 670

AAG AAG TAC TCC AAG CAG GTG GAT GAC CGA TTC GGT 2199
Lys Lys Tyr Ser Lys Gln Val Asp Asp Arg Phe Gly
        675                 680

GCC TAC GTG GCA TGT GCC TCG CTT GTC TTC CTC TTC     2235
Ala Tyr Val Ala Cys Ala Ser Leu Val Phe Leu Phe
685                 690                 695

ATC TGC TTT GTC CAG ATC ACC ATC GTA CCC CAC TCC     2271
Ile Cys Phe Val Gln Ile Thr Ile Val Pro His Ser
            700                 705

GTG TTC ATG TTG AGT TTC TAC TTG ACC TGT TTC CTG     2307
Val Phe Met Leu Ser Phe Tyr Leu Thr Cys Phe Leu
    710                 715                 720

CTG CTG ACG TTG GTG GTA TTT GTG TCC GTG ATC TAT     2343
Leu Leu Thr Leu Val Val Phe Val Ser Val Ile Tyr
                725                 730

TCC TGC GTG AAG CTC TTC CCG GGC CCG CTC CAG AGC     2379
Ser Cys Val Lys Leu Phe Pro Gly Pro Leu Gln Ser
        735                 740

CTC TCG AGG AAG ATC GTG CGC TCC AAG ACC AAC AGC     2415
Leu Ser Arg Lys Ile Val Arg Ser Lys Thr Asn Ser
745                 750                 755

ACC CTG GTC GGG GTG TTC ACC ATC ACC CTG GTG TTC     2451
Thr Leu Val Gly Val Phe Thr Ile Thr Leu Val Phe
            760                 765

CTG TCG GCT TTC GTC AAC ATG TTC ATG TGT AAC TCC     2487
Leu Ser Ala Phe Val Asn Met Phe Met Cys Asn Ser
    770                 775                 780

GAG GAC CTG TTG GGC TGC CTG GCG GAC GAG CAC AAC     2523
Glu Asp Leu Leu Gly Cys Leu Ala Asp Glu His Asn
                785                 790

ATC AGC ACC AGC CGG GTC AAC GCG TGC CAC GTG GCG 2559
Ile Ser Thr Ser Arg Val Asn Ala Cys His Val Ala
        795                 800

GCG TCG GCG GCC AAC CTC AGC CTG GGC GAC GAG CAG 2595
Ala Ser Ala Ala Asn Leu Ser Leu Gly Asp Glu Gln
805                 810                 815

GGC TTC TGC GGC ACG CCC TGG CCC AGC TGC AAC TTC 2631
Gly Phe Cys Gly Thr Pro Trp Pro Ser Cys Asn Phe
            820                 825

CCC GAG TAC TTC ACC TAC AGC GTG CTG CTC AGC CTG 2667
Pro Glu Tyr Phe Thr Tyr Ser Val Leu Leu Ser Leu
    830                 835                 840

CTG GCC TGC TCC GTG TTC CTG CAG ATC AGC TGC ATC 2703
Leu Ala Cys Ser Val Phe Leu Gln Ile Ser Cys Ile
                845                 850

GGG AAG CTG GTG CTC ATG CTG GCC ATT GAG CTC ATA 2739
Gly Lys Leu Val Leu Met Leu Ala Ile Glu Leu Ile
        855                 860

TAC GTG CTC GTC GTC GAG GTG CCC CGG GTC ACA CTG 2775
Tyr Val Leu Val Val Glu Val Pro Arg Val Tyr Leu
865                 870                 875

TTT GAC AAC GCT GAC CTG CTG GTC ACC GCC AAC GCC 2811
Phe Asp Asn Ala Asp Leu Leu Val Thr Ala Asn Ala
            880                 885

ATA GAC TTC AAC AAC AAC AAC GGG ACC TCG CAG TGC 2847
Ile Asp Phe Asn Asn Asn Asn Gly Thr Ser Gln Cys
    890                 895                 900

CCT GAG CAC GCG ACC AAG GTG GCG CTG AAG GTG GTG 2883
Pro Glu His Ala Thr Lys Val Ala Leu Lys Val Val
                905                 910

ACG CCC ATC ATC ATC TCC GTC TTC GTG CTG GCC CTG 2919
Thr Pro Ile Ile Ile Ser Val Phe Val Leu Ala Leu
        915                 920

TAC CTG CAT GCC CAG CAA GTG GAG TCC ACC GCC CGC 2955
Tyr Leu His Ala Gln Gln Val Asp Ser Thr Ala Arg
925                 930                 935

CTC GAC TTC CTC TGG AAA CTG CAG GCC ACG GAG GAG 2991
Leu Asp Phe Leu Trp Lys Leu Gln Ala Thr Glu Glu
            940                 945

AAG GAG GAG ATG GAG GAG CTG CAG GCC TAC AAC CGG 3027
Lys Glu Glu Met Glu Glu Leu Gln Ala Tyr Asn Arg
    950                 955                 960

CGG CTG CTG CAC AAC ATC CTG CCC AAG GAC GTG GCT 3063
Arg Leu Leu His Asn Ile Leu Pro Lys Asp Val Ala
                965                 970

GCC CAC TTC CTG GCC CGT GAG CGA CGC AAC GAC GAG 3099
Ala His Phe Leu Ala Arg Glu Arg Arg Asn Asp Glu
        975                 980

CTC TAC TAC CAG TCG TGC GAG TGC GTG GCT GTC ATG 3135
Leu Tyr Tyr Gln Ser Cys Glu Cys Val Ala Val Met
985                 990                 995

TTC GCC TCC ATC GCC AAC TTC TCC GAG TTC TAC GTG 3171
Phe Ala Ser Ile Ala Asn Phe Ser Glu Phe Tyr Val
            1000                1005

GAG CTT GAG GCC AAC AAT GAG GGT GTC GAG TGC CTG 3207
Glu Leu Glu Ala Asn Asn Glu Gly Val Glu Cys Leu
    1010                1015                1020

CGC GTG CTC AAT GAG ATC ATC GCT GAC TTT GAT GAG 3243
Arg Val Leu Asn Glu Ile Ile Ala Asp Phe Asp Glu
                1025                1030

ATC ATC AGC GAG GAT CGG TTC AGG CAG CTG GAG AAG 3279
Ile Ile Ser Glu Asp Arg Phe Arg Gln Leu Glu Lys
        1035                1040

ATC AAG ACC ATT GGC AGC ACC TAC ATG GCC GCC TCA 3315
Ile Lys Thr Ile Gly Ser Thr Tyr Met Ala Ala Ser
1045                1050                1055

GGC CTC AAT GAC TCT ACA TAC GAC AAG GTG GGC AAG 3351
Gly Leu Asn Asp Ser Thr Tyr Asp Lys Val Gly Lys
            1060                1065

ACC CAC ATC AAA GCC CTG GCT GAC TTT GCC ATG AAG 3387
Thr His Ile Lys Ala Leu Ala Asp Phe Ala Met Lys
    1070                1075                1080

CTC ATG GAC CAA ATG AAG TAC ATC AAT GAG CAC TCC 3423
Leu Met Asp Gln Met Lys Tyr Ile Asn Glu His Ser
                1085                1090

TTC AAC AAC TTC CAG ATG AAG ATC GGG CTC AAC ATC 3459
Phe Asn Asn Phe Gln Met Lys Ile Gly Leu Asn Ile
        1095                1100

GGC CCC GTG GTG GCC GGG GTG ATC GGG GCT CGC AAG 3495
Gly Pro Val Val Ala Gly Val Ile Gly Ala Arg Lys
1105                1110                1115

CCT CAG TAC GAC ATC TGG GGC AAT ACG GTG AAT GTG 3531
Pro Gln Tyr Asp Ile Trp Gly Asn Thr Val Asn Val
            1120                1125

GCC AGC CGC ATG GAC AGC ACC GGC GTG CCG GAC CGC 3567
Ala Ser Arg Met Asp Ser Thr Gly Val Pro Asp Arg
    1130                1135                1140

ATC CAG GTC ACC ACG GAC ATG TAC CAG GTG TTG GCT 3603
Ile Gln Val Thr Thr Asp Met Tyr Gln Val Leu Ala
                1145                1150

GCC AAC ACG TAC CAG CTG GAG TGC AGG GGT GTG GTC 3639
Ala Asn Thr Tyr Gln Leu Glu Cys Arg Gly Val Val
        1155                1160

AAG GTC AAG GGC AAA GGC GAG ATG ATG ACC TAC TTC 3675
Lys Val Lys Gly Lys Gly Glu Met Met Thr Tyr Phe
1165                1170                1175

CTC AAC GGT GGG CCC CCG CTC AGT 3699
Leu Asn Gly Gly Pro Pro Leu Ser
1180 1184

TAGCACCTGC CAACTGGTGA TGCCAGGCCG CCTGGCCTCC 3739

TGAGAAACGG AAATGGCTTC TTTGTGTGCC GGTGGGCAGG 3779

TGGAAGCCTG CGCTCTAGCC CGTGTGGCCG CGCCGGTGAG 3819

ATTTTCCACT TTGACTCCAG AAGCAGCTTC TGCCTTTGTG 3859

GGCAGCCCAG GCCCTGGGGC GCCAGCGTCC TGCGAGCACC 3899

AAGCTGAACA AAGATGTTTC CTCCGTGGAA GACTCCGCCA 3939

GGCGTGATCT GAGTCTCGAG TTTTCTAACG GGTGCTGCTA 3979

CTGCACGGGT AGAAAGGAGC TGCTGGAGCT CTGGCGGCGT 4019

GTGGTAGCCA GGCGTGCGGC GACAGGGCTG ACGGGGAGGC 4059

CTCGGCTGGG GGACCCAGGG CCCCGCTTTC CTGTGTGAGC 4099

ATTTCAGCTT CCGCCAGAGC GCACGGCCCC TGCCCCACGG 4139

AGGCGTTCTG GCGGGAGTGC TGGCTGTGGA GGGGCTGCGG 4179

CTTCTCGCAG TCCTCCTCCT GCACCCGCAC ACGTAGACGA 4219

CGCCTCGTCG AGGGGAACGG AACCAAGTGC GAGGGGGAGG 4259

CGAGAGGACT CGAGGCAAGG AGGGGTGGTT CTGAGAAAAA 4299

GAATATTTAT TAAATAAAAC AACTTTCTGT GCCCTTAAAA 4339

AAAAAAAAAA AAAAAAA 4356

## Description

### BACKGROUND OF THE INVENTION

This application is a continuation-in-part of U.S. Serial No. 751,469, filed August 29, 1991, the contents of which are hereby incorporated.

Throughout this application, various publications are referred to by an arabic numeral within parentheses. Full bibliographic citation for each reference may be found at the end of the specification, immediately preceding the sequence listing. The disclosures of these citations describe the state of the art to which this invention pertains and are hereby incorporated by reference into the present disclosure.

The signal transduction pathway may be subdivided into three steps. The first is the recognition of the ligand by the receptor. The second is the transmission and amplification of the signal by a "transducer" protein. The final step is the generation of the second messenger by an effector enzyme.

Adenylyl cyclases are effector enzymes that are coupled to various hormone-receptor systems, such as catecholamine and ACTH. The catecholamine receptor and its transducer protein (G-protein) have been well characterized since the cloning of their cDNAs. However, relatively little is known about the adenylyl cyclase.

Once such a hormone binds to the receptor, it activates G protein, a heterotrimeric guanine nucleotide-binding regulatory protein (α, β, γ). The activated G-protein elicits the exchange of GDP for GTP, as well as the dissociation from βγ subunits. The GTP bound form of the α-subunit stimulates adenylyl cyclase, which generates cyclic AMP from ATP. Cyclic AMP, a second messenger, activates various proteins, including protein kinases.

Protein kinases then phosphorylate other proteins, thus initiating a signal transduction cascade. Another type of activation is through the increased intracellular calcium concentration, especially in nervous tissues. After depolarization, the influx of calcium elicits the activation of calmodulin, an intracellular calcium binding protein. The activated calmodulin has been shown to bind and activate an adenylyl cyclase directly (1).

Several papers have suggested the diversity of the adenylyl cyclases. Using forskolin-bound affinity chromatography, a single class of the enzyme protein was purified from bovine brain (2,3). The monoclonal antibody raised against this purified protein also recognized another form of protein in the brain, which was different in size. Biochemical characteristics have shown that these two are different types of adenylyl cyclase; one is calmoduline-sensitive (CaM-sensitive) and the other is CaM-insensitive. This study (2) showed that there are two types of adenylyl cyclase in one tissue, and that these types share the same domain that could be recognized by the same antibody.

Another paper has presented genetic evidence of the diversity of adenylyl cyclase (4). An X-linked recessive mutation in Drosophilla which blocked associative learning lacked the CaM-sensitivity of adenylyl cyclase, but did possess the reactivity to fluoride or GTP. This suggests that the CaM-sensitive cyclase gene is located in the X-chromosome, which is distinct from the CaM-insensitive adenylyl cyclase gene.

Three different cDNAs have been cloned from mammalian tissues so far. These have been designated type I (brain type (5)); II (lung type (6)); and III (olfactory type (7)). The cDNA sequences of Types I and III have been published. The adenylyl cyclases coded for by these cDNAs are large proteins more than 1000 amino acids in length. Topographically, all types are similar. All have two six-transmembrane domains associated with a large cytoplasmic loop. The amino acid sequence of the cytoplasmic loop is conserved among different types of cyclase.

Tissue distribution of these adenylyl cyclase messages is well distinguished, as shown in Northern blotting studies. Type I is expressed only in the brain, type II is distributed in lung and brain, and type III is expressed mostly in the olfactory tissue with little expression in the brain. Thus, the adenylyl cyclases are distributed in a rather tissue specific manner. Despite the fact that heart tissue was one of the tissues in which adenylyl cyclase was originally identified, none of the three known types has been shown to be expressed in heart tissue.

It has been documented that a form of adenylyl cyclase is also present in the heart (8), and that the cyclase from the heart is recognized by a monoclonal antibody originally raised against the cyclase from the brain (9). Given that the three cloned types of adenylyl cyclase have a conserved amino acid sequence in their large cytoplasmic loop, the cyclase from the heart may share sequence homology in this region. Thus, it is possible to attempt to obtain an adenylyl cyclase clone from the heart by using an adenylyl cyclase cDNA from the brain. However, no adenylyl cyclase has been reported to have been cloned from cardiac tissue or expressed.

### Summary of the Invention

This invention provides an isolated nucleic acid molecule encoding a cardiac adenylyl cyclase type V and the polypeptide encoded by the nucleic acid molecule. Methods of screening for drugs and modifying cardiac function also are provided.

### Brief Description of the Figures

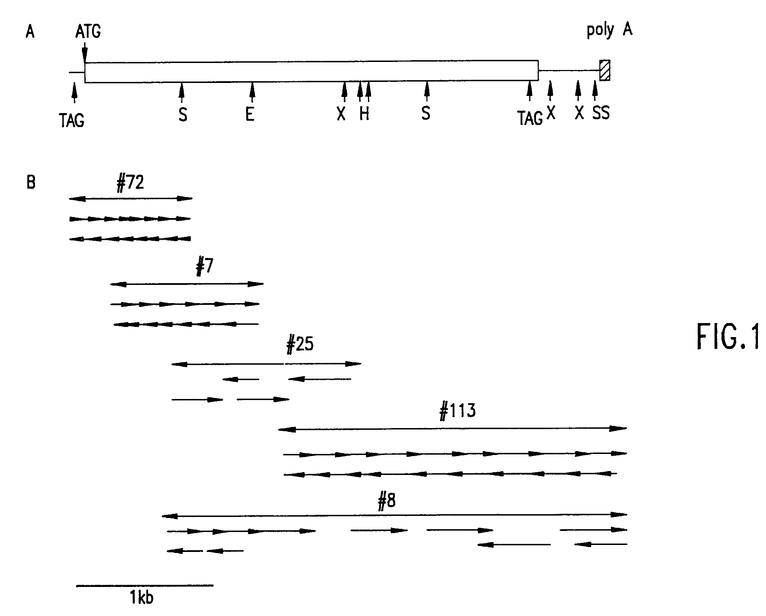

Figure 1A depicts partial restriction map of Type V adenylyl cyclase: A: partial restriction map of adenylyl cyclase cDNA. Coding portion is boxed and the hatched box shows the polyadenylyl site. E: EcoRI restriction site. H: HincII. S: Sph1X. X: XhoI. SS: SspL.

Figure 1B depicts cDNA clones of Type V adenylyl cyclase obtained from canine heart λgt10 library and the sequencing strategy. Five different clones, #7, #8, #25, #72 and #113, ranging in size from 1.2 to 3.5 kb, constitute a 4.4-kb cDNA, which contains the entire coding region.

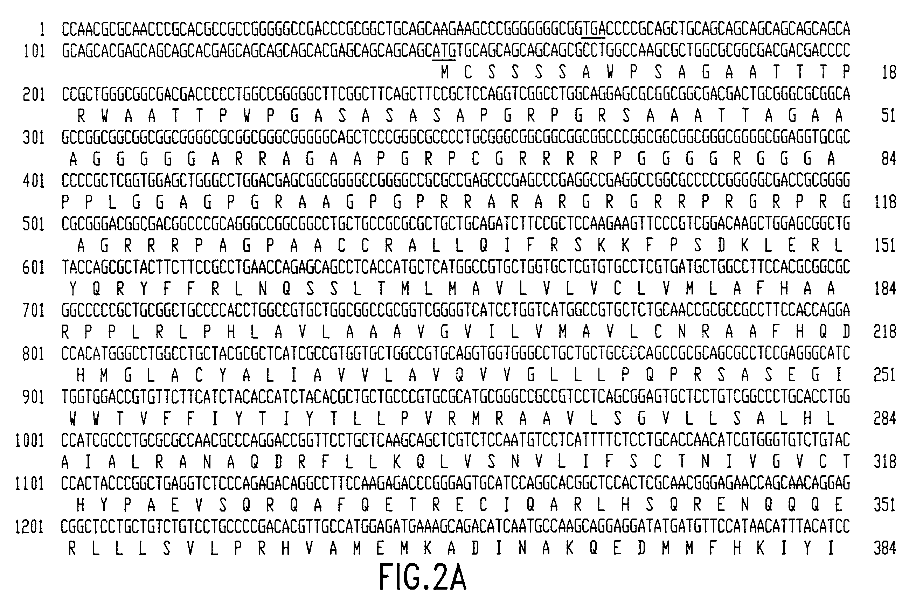

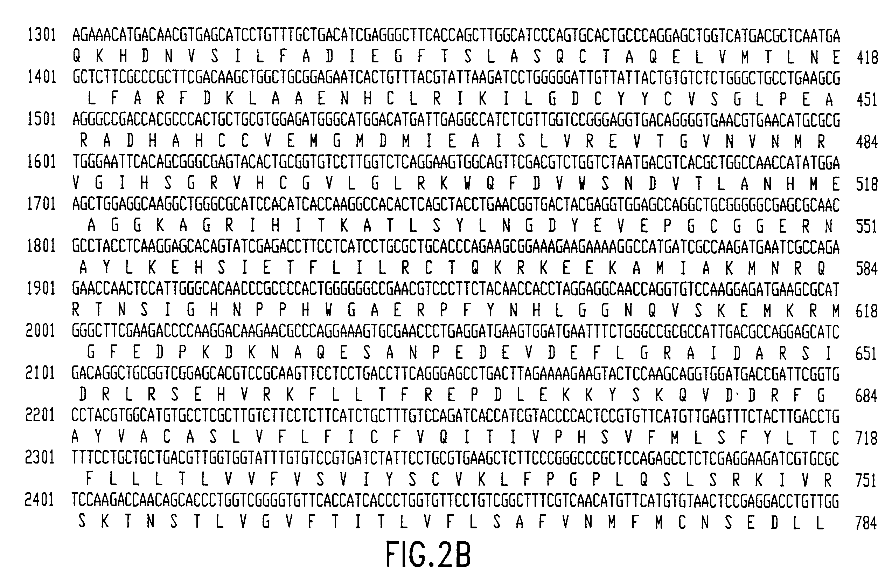

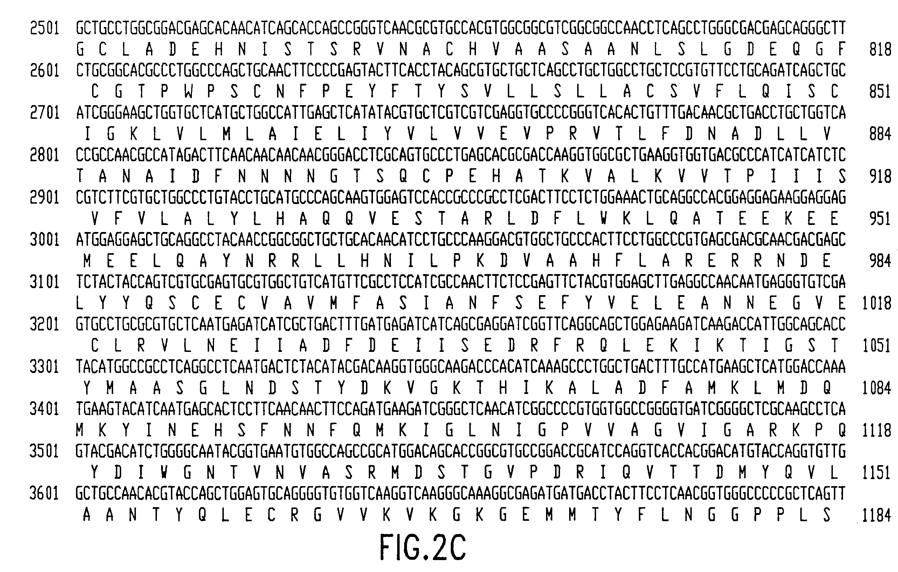

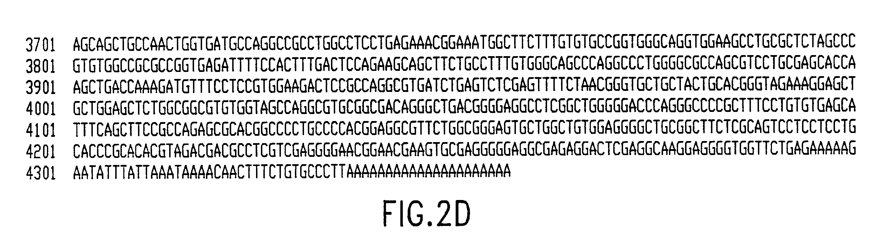

Figure 2 depicts the DNA and predicted amino acid sequence of the cardiac adenylyl cyclase. The entire coding sequence, as well as a portion of the 5' untranslated and complete 3' untranslated sequences, are shown. ATG shows the putative translation initiation site in the open reading frame. TGA shows the translation termination codon in the open reading frame. The putative polyadenylylation signed is marked in italic (AATAAA). Also shown as Sequence ID #1.

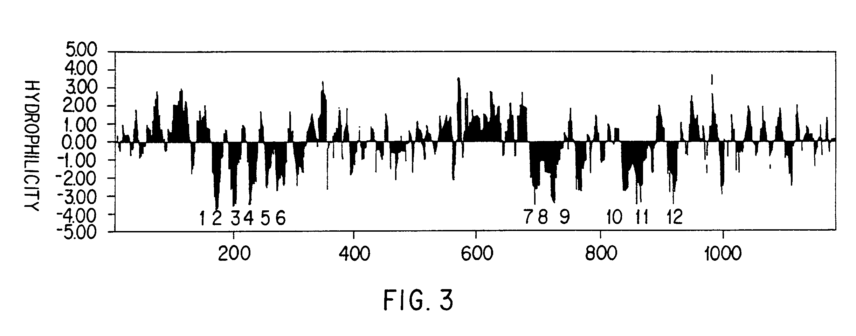

Figure 3 is a hydropathy plot of the Type V adenylyl cyclase and protein dot matrix comparison with Type I adenylyl cyclase. MacVector 3.5 software was used to analyze the structure of Type V adenylyl cyclase. The method of Kyte and Doolittle (11) is used with a window size of 7. Twelve peaks are numbered which represent putative transmembrane spanning regions.

Figure 4A is as in Figure 3, but MacVector 3.0 is used for the amino acid dot matrix comparison between type V and type I with the stringency set at 60% and the window size at 8.

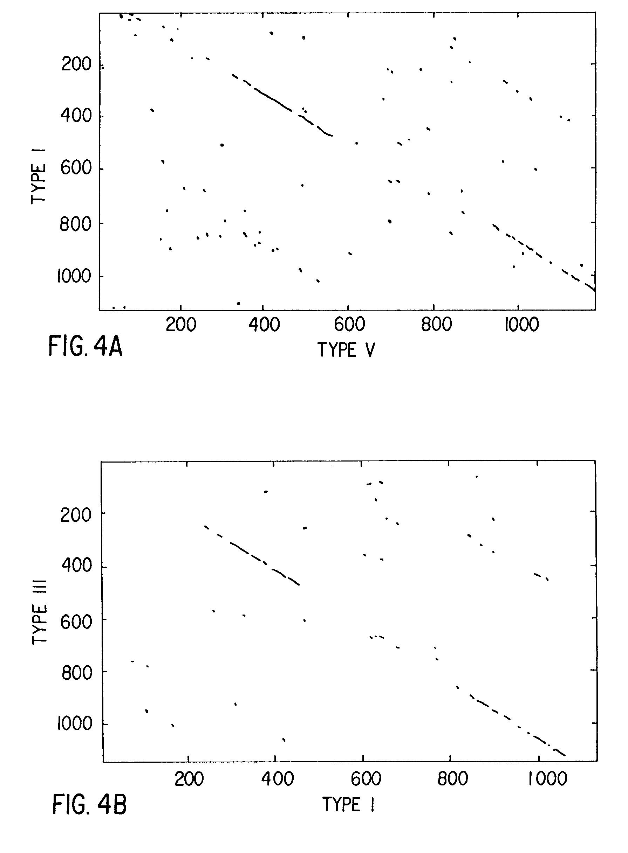

Figure 4B depicts a protein dot matrix comparison between other types of adenylyl cyclases (type I and type III). MacVector 3.0 software is used for the analysis with a stringency of 65% and a window size of 8.

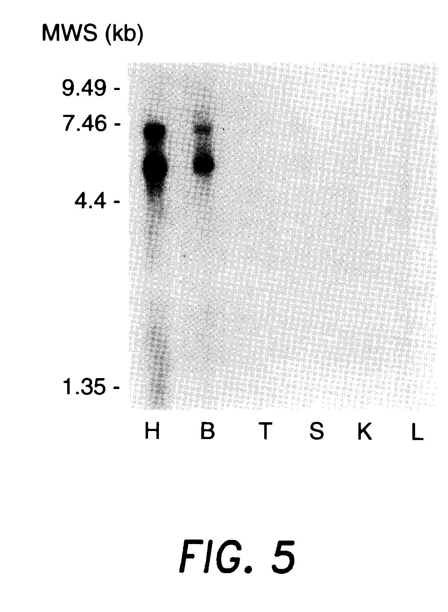

Figure 5 depicts Northern blot analysis of various canine tissues by a fragment from cardiac adenylyl cyclase cDNA. Five µg of poly(A) RNA are employed for each assay. An EcoRI-HincII 0.9 kb fragment from the cardiac adenylyl cyclase cDNA is used as a probe. The lanes are as follows: H-heart, B-brain, T-testis, S-skeletal muscle, K-kidney, L-lung. Standards in kilobases (kb) are at the left of the blot.

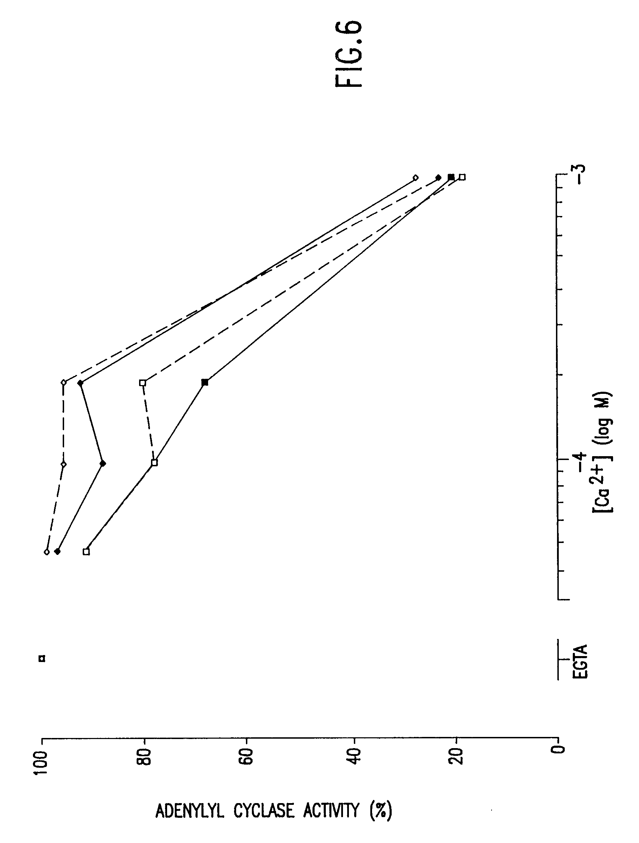

Figure 6 depicts the effect of calcium on type V adenylyl cyclase activity. Adenylyl cyclase activity (CMT cells transfected with pcDNA113-72 or cardiac sarcolemma) is measured in the presence of increasig concentrations of calcium (0-1 mM). Cardiac sarcolemma is prepared as described (26). Both membrane preparations are first washed with EGTA prior to assay (27). Similar results are obtained in three independent experiments. The efficiency of transfection is confirmed by at least a 4-fold increase in both basal and forskolin-stimulated adenylyl cyclase activities over control. Transfected membrane (□, transfected membrane with calmodulin (200 nM) (■), cardiac Sarcolemma (◇), cardiac sarcolemma with calmodulin (200 nM) (♢].

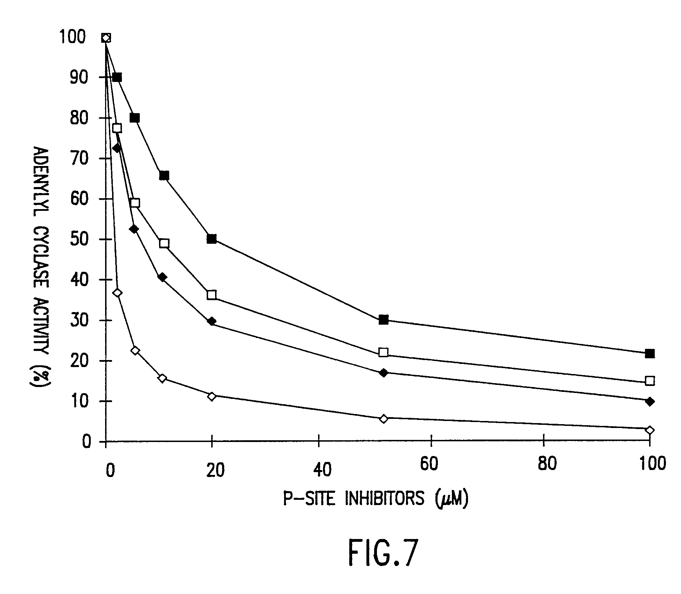

Figure 7 depicts the effect of adenosine and its analogues on type V adenylyl cyclase activity. Membrane (transfected with pcDNA112-72 or control) are preincubated with 5 mM Mn²⁺ and 100 µM forskolin for 10 minutes prior to the assay. Adenosine (■), 2'-deoxyadenosine (□), 3'-AMP (♢), and 2'deoxy-3'-AMP (◇). similar results are obtained in three independent experiments. Each point in the average of triplicate determinations.

### Detailed Description of the Invention

This invention provides an isolated nucleic acid molecule encoding a cardiac adenylyl cyclase type V polypeptide. As used herein, the term "nucleic acid" encompasses RNA as well as single and double-stranded DNA and cDNA. In addition, as used herein, the term "polypeptide" is intended to mean a linear polymer of amino acids linked by means of peptide bonds and encompasses any naturally occurring allelic variant thereof as well as man-made recombinant forms. The cardiac adenylyl cyclase type V polypeptide is preferably a mammalian polypeptide, e.g., canine or human. In the most preferred embodiment, the polypeptide is human polypeptide.

Examples of such nucleic acids include, but are not limited to the nucleic acids shown in Sequence I.D. Nos. 1 and 2. This invention also encompasses nucleic acid molecules which differ from that of the nucleic acid molecules which encode these amino acid sequences, but which produce the same phenotypic effect. These altered, but phenotypically equivalent nucleic acid molecules are referred to as "equivalent nucleic acids". And this invention encompasses nucleic acid molecules characterized by changes in non-coding regions that do not alter the phenotype of the polypeptide produced therefrom when compared to the nucleic acid molecule described hereinabove. This invention further encompasses nucleic acid molecules which hybridize to the nucleic acid molecules shown in Sequence I.D. Nos. 1 and 2.

As noted above, adenylyl cyclase type V polypeptide encompasses naturally occurring, synthetic and recombinant forms, i.e., non-naturally occurring forms of the polypeptide which are sufficiently identical to naturally occurring polypeptides to allow possession of similar biological activity. Such polypeptides include derivatives and analogs.

Also provided by this invention is a purified mammalian polypeptide corresponding to an adenylyl cyclase type V polypeptide, wherein the purified polypeptide is glycosylated. However, this invention also encompasses unglycosylated forms of the polypeptide and purified mammalian polypeptides having glycosylation sufficiently similar to that of naturally occurring purified mammalian adenylyl cyclase type V polypeptide.

Also provided by this invention is a pharmaceutical composition which comprises an effective amount of the purified mammalian polypeptide described hereinabove or antibody described below and a pharmaceutically acceptable carrier. The pharmaceutical compositions of this invention are useful in therapy or in diagnostic assays. As used herein, the term "pharmaceutically acceptable carrier" encompasses any of the standard pharmaceutical carriers, such as phosphate buffered saline solution, water, emulsions, such as oil/water emulsions, and various types of wetting agents.

Also provided by this invention is a vector which comprises the nucleic acid molecule which encodes an amino acid sequence corresponding to an adenylyl cyclase type V polypeptide. This vector includes, but is not limited to a plasmid, viral or cosmid vector.

This invention also provides the isolated nucleic acid molecule of this invention operatively linked to a promoter of RNA transcription, as well as other regulatory sequences. As used herein, the term "operatively linked" means positioned in such a manner that the promoter directs the transcription of RNA off of the nucleic acid molecule. Examples of such promoters are SP6, T4 and T7. Vectors which contain both a promoter and a cloning site into which an inserted piece of DNA is operatively linked to that promoter are well known in the art. Preferably, these vectors are capable of transcribing RNA in vivo or in vitro.

A host vector system for the production of the adenylyl cyclase type V polypeptide is further provided by this invention which comprises one of the vectors described hereinabove in a suitable host. For the purposes of this invention, a suitable host is, but is not limited to, a eucaryotic cell, e.g., a mammalian cell, a yeast cell or an insect cell for baculovirus expression. The suitable host is also a procaryotic cell such as a bacteria cell, e.g., E. coli.

A method for biosynthetically producing an amino acid which corresponds to an adenylyl cyclase type V polypeptide is also provided which comprises growing the host vector system described hereinabove under suitable conditions permitting production of the adenylyl cyclase type V and recovering the resulting adenylyl cyclase type V. This invention also provides the adenylyl cyclase type V produced by this method.

This invention further provides a substance capable of specifically forming a complex with the adenylyl cyclase type V polypeptide, or a fragment thereof, described hereinabove. In one embodiment of this invention, the substance is an antibody, preferably a monoclonal antibody, e.g., a mouse or human monoclonal antibody.

Further provided are pharmaceutical compositions comprising the monoclonal antibody described hereinabove alone, or conjugated to a detectable marker such as a radioisotope. By marker is intended a moiety which provides directly or indirectly, a detectable signal. Various markers are employed, such as radioisotopes, enzymes, fluorescers, chemiluminescers, and the like. For the purposes of this invention, suitable radioisotopes include, but are not limited to, ³²P, ³⁵S and ¹³¹I.

For the isolation of mouse monoclonal antibodies, eight week old mice are injected intraperitoneally with about 50 micrograms of a polypeptide (prepared as described above) in complete Freund's adjuvant 1:1 volume. Mice are boosted, at monthly intervals, with the polypeptide, mixed with incomplete Freund's adjuvant, and bled through the tail vein. On days 4, 2 and 2 prior to fusion, mice are boosted intravenously with 50 micrograms of the polypeptide in saline. Splenocytes are then fused with nonsecreting myeloma cells according to procedures which have been described and are known to those of ordinary skill in the art to which this invention pertains. Some time later, approximately two weeks later, hybridoma supernatant is screened for binding activity against the polypeptide.

Isolation of human monoclonal antibodies is similar except human β-lymphocyte cells are isolated from patients and transformed with EBV. The β-lymphocyte cells are fused with non-secreting myeloma cells according to procedures which have been described and are known to those of ordinary skill in the art to which this invention pertains. Some time later, approximately two weeks later, hybridoma supernatant is screened for binding activity against the polypeptide. Positive clones are isolated and propagated. Alternatively, human monoclonal antibodies are prepared using methods described in Patent Nos. 4,720,459, 4,693,975 and 4,574,115, the contents of which are hereby incorporated by reference.

Cardiac function in a patient is modified by administering to the patient the polypeptide of this invention alone or in a pharmaceutical composition. "Administering" means a method of administering to the patient. Such methods are well known to those skilled in the art and include, but are not limited to administration orally, intravenously, or parenterally. Administration of the agent is effected continuously or intermittently, such that the amount of the polypeptide in the patient is effective to modify the cardiac function.

Cardiac function in a subject, such as a human patient is determined by isolating a suitable RNA sample from the subject sample, and hybridizing the cDNA of this invention to the RNA is the sample. The methods of isolating and determining the amount of RNA in the sample are well known to those of skill in the art (16).

The strategy used to identify and isolate the novel cardiac adenylyl cyclase begins with the construction and screening of canine heart cDNA library.

Left ventricular tissue of canine heart is used as a source of mRNA. The library is prepared in a λgt10 phage with an oligo-dT primer as described (12). The primary screening of the λgt10 library is carried out in less stringent hybridization and washing conditions. Approximately 2 x 10⁶ plaques are initially screened from the library. Prehybridization is carried out for at least two hours in a solution containing 30% formamide, 5 x SSC, 5 x Denhardt's, 25 mM NaPO₄ (pH 6.5), 0.25 mg/ml calf thymus DNA, and 0.1% sodium dodecyl sulfate (SDS) at 42^{o}C. Hybridization is then performed in the same solution at 42^{o}C. A 970 base pair (bp) AatI-HincII fragment from type I adenylyl cyclase cDNA is used as a probe. This fragment encodes the first cytoplasmic domain of the adenylyl cyclase, which has significant homology to other previously-known types of adenylyl cyclase (7).

The probe is radiolabelled with ³²P-dCTP by the multi-primer-random labelling method. After hybridization for 18 hours, the blot is washed under increasingly stringent conditions and then radioautographed. Five positive clones are obtained. The sizes of the inserts in the clones range from 0.7 kb (kilo-bases) to 3.5 kb.

The next step is to ascertain the full length cDNA sequence from the inserts in the clones. All the positive clones from the canine heart library are subcloned into plasmid pUC18. After restriction maps are made, they are further subcloned and sequenced with universal primers or synthesized oligomers. For some fragments, sequencing is performed after a series of deletions is made by exonuclease III digestion. The sequence is performed bidirectionally at least twice with either Sequenase (13) or by Taq polymerase (14). In some GC-rich areas, the sequence is performed using a gel containing 7% polyacrylamide, 8 M urea, and 20% formamide.

Three clones, named #8, #25 and #113, are identified to be overlapping with each other over a length of 3.5 kb (Figure 1). After the whole inserts are sequenced, it is found that clones 8 and 13 use the same polyadenylation site. These two fragments make up 3.5 kb of a cDNA fragment. However, this 3.5 kb fragment did not contain an initiator ATG with an optimal Kozak consensus sequence in the long open reading frame. In order to obtain the 5' end of this cDNA, an 800-bp 5' EcoRI fragment from clone 8 is used as a probe to screen the λgt10 library. After sequencing the entire set of new cDNA clones, clone 7 overlapped for 800 bases with clone 8 and extended the sequence upstream an additional 400 bp. To obtain the additional 5' sequence, fragments consisting of either the most upstream 60 or 500 bp of clone 7 are used to rescreen the cDNA library. Out of 82 primary positives clone 72 is obtained, which extended the sequence an additional 380 bp. The restriction map of these clones is shown in Figure 1. These clones (7 and 72) contain highly GC-rich regions. Sequencing is performed bidirectionally at least three times using both Sequenase and Taq polymerase with or without 7-deaza-dGTP. The reaction mixtures are run in a polyacrylamide gel containing both 8 M urea and 20% formamide.

A putative ATG translation initiation codon, which exists in the context of a reasonable Kozak consensus sequence (15), is in an extended open reading frame. A stop codon (TGA) 81 bp upstream of this ATG is in the same open reading frame (Figure 2). This region is highly GC-rich as seen in other types of adenylyl cyclase cDNAs (2-5). ATG initiates the translation of an open reading frame of 3552 bases, encoding a 1184-amino acid protein, followed by 634 bp of a 3'-untranslated region upstream of the polyadenylation site. The homology to brain adenylyl cyclase (type I) is higher in the cytoplasmic but lower in the transmembrane portion as shown by the dot matrix comparison (Figure 3B). The first cytoplasmic domain, especially the 5' half of the loop, is highly homologous to other types of cyclase including yeast adenylyl cyclases (24) and various types of guanylyl cyclase (25, 26). This suggests the presence of an essential function within this domain, e.g., ATP binding. The extracellular loop between the 9th and 10th transmembrane-spanning regions is the largest (Figure 3A). In comparison to other types of mammalian adenylyl cyclases, type V is more similar to types II and IV in that it has a shorter C-terminal tail, but is unique among the cyclases in having a much longer N-terminal tail (type V, 164 amino acids; type I, 63; type II, 44; type III, 77; and type IV, 28).

The tissue distribution of this novel gene product is examined by Northern blotting using a probe unique to this cDNA, so tissues known to possess high adenylyl cyclase activity are examined. The message is most abundantly expressed in the heart, to a lesser degree in the brain, while no expression is detected in other tissues (testis, skeletal muscle, kidney and lung). Both heart and brain contained messages of ∼5 and 7 kb (Figure 4). The ratio (3:2) of these mRNAs was similar between the two tissues. When different probes from different portions of the cDNA are used, i.e., the transmembrane portion, the entire cDNA, or 3'-untranslated portion, the Northern blotting results are similar. Thus far six type V adenylyl cyclase clones are obtained which contain a complete 3' end. There is no divergence in their sequence and all used the same polyadenylation site. Taken together, these findings suggest that the two messages are most likely products of the same gene, probably the result of alternative splicing of a single precursor RNA. The message sizes predict that each contains at least 1-3 kb of 5'-untranslated sequences. The expression of this message in several cell lines also is examined. Messenger RNA of similar size is detected in GH₄ and PC12 cells but not in S49 or BAEC. It is of interest that when a probe from the second cytoplasmic portion of this cDNA is employed, i.e., a 1.8-kb XhoI fragment from clone 113, which shares homology with other types of adenylyl cyclase, an RNA species of approximately 6 kb is also seen in poly(A)⁺ RNA prepared from heart and brain if the hybridization is carried out under relatively less stringent conditions. A much larger species is detected in skeletal muscle under similar conditions. The size of this message is approximately 9.5 kb. These additional mRNAs might represent other members of the adenylyl cyclase family not yet identified.

Biochemical characterization of the protein product encoded by this cDNA is obtained using a CMT cell-expression system. The CMT cell is a derivative of the COS cell in which expression of the T-antigen is under the control of a metallothionein promoter. Thus the accumulation of T-antigen in the cell is further enhanced by the addition of heavy metal ion in the medium. The adenylyl cyclase cDNA construct (113-72) is cloned into pcDNA 1, a CMV promoter-driven expression vector with an SV40 enhancer-origin of replication element. A crude membrane preparation is prepared from the transfected CMT cells and a variety of agents known to stimulate adenylyl cyclase are examined (Table I). There is a dose-dependent relationship between the amount of plasmid transfected and the resultant adenylyl cyclase activity, both basal and forskolin-stimulated (0-30 µg per transfection). When the plasmid without insert is transfected, the resultant adenylyl cyclase activity is slightly (∼15%) lower than that of mock-transfected cells. All activities are enhanced several-fold in the transfected cells, as compared to controls, with the forskolin-stimulated activity showing the greatest increase in activity (>6-fold increase over control).

The calcium/calmodulin sensitivity of this protein is also assessed. Adenylyl cyclase purified from heart has previously been shown to be inhibited by the addition of calcium (27). Thirty µg of the crude CMT cell membrane preparation are incubated in the presence of increasing concentrations of CaCl₂. The transfected adenylyl cyclase in the CMT cells is inhibited in a concentration-dependent manner by the addition of 0-1 mM calcium. Addition of calmodulin (200 mM) did not alter this inhibitory effect. The adenylyl cyclase activity also is assessed in canine cardiac sarcolemma. It exhibits a similar degree of inhibition in the presence of calcium (Figure 6).

Another feature of adenylyl cyclase is its inhibition through an allosteric purine binding or P-site (6). Adenylyl cyclase activity in the transfected CMT membranes exhibits a concentration-dependent inhibition in the presence of adenosine and its analogues (0-100 µM). This is more apparent when the enzyme is first activated by the addition of Mn²⁺, forskolin. The order of potency was as follows: 2'-deoxy-3'-AMD>3'-AMP>2'-deoxyadenosine>adenosine (Figure 7). The above data indicate that the protein encoded by this cDNA is adenylyl cyclase with the biochemical features of the cardiac isoform.

The combined cDNA fragments are subcloned into pcDNA1 (Invitrogen, San Diego, CA), a mammalian expression vector with a CMV promoter and SV40 enhancer elements, using the unique restriction sites. The expression vector containing the full length cDNA is designated pcDNA113-72. Samples of this expression vector, inserted into an appropriate E. coli cell line designated MC1061/P3, have been deposited with the American Type Culture Collection, 12301 Parklawn Drive, Rockville, Maryland 20852, in accordance with the Budapest Treaty on the International Recognition of the Deposit of Microorganisms for the Purposes of Patent Procedure and have been accorded accession number ATCC 68888.

A nucleic acid sequence encoding human type V adenylyl cyclase is isolated from a human cardiac cDNA library. A canine type V adenylyl cyclase cDNA clone, #113, is used as a probe to screen 2.0 x 10⁶ independent clones. The hybridization is carried out in a solution containing 50% formamide, 5x SSC, 5x Denhardt's, 25 mM NaPO₄ (pH 6.5), 0.25 mg/ml calf thymus DNA and 0.1% SDS at 42^{o}C. Hybridization is carried out in the same buffer at 42^{o}C for 14-20 hours with cDNA fragments labeled with ³²P, followed by washing under increasingly stringent conditions. All positive clones from the human heart library are subcloned into pUC18. After restriction mapping, the fragments are subcloned and sequenced with universal primers. A clone, #341, contains an insert of 1.4 kb, whose sequence is highly homologous to canine type V adenylyl cyclase. Indeed, the homology is over 90% in the region sequenced (∼0.3 kb). DNA dot blot matrix comparisons showed the clone #341 coding the region equivalent to that of canine type V adenylyl cyclase cDNA from the nucleotide residue 1902 to 4328. This region in the canine isoform encodes the latter half of the first cytoplasmic domain, which shows a high variability among different types of adenylyl cyclase, indicating the clone #341 is a human analog of canine type V adenylyl cyclase.

Clone #341 has been deposited with the American Cype Culture Collection (ATCC), 12301 Parklawn Drive, Rockville, Maryland 20852, under the provisions of the Budapest Treaty on the International Recognition of the Deposit of Microorganisms for the Purposes of Patent Procedure and has been accorded Accession Number .

Production of this cardiac adenylyl cyclase is achieved by the cloning and expression of the cardiac adenylyl cyclase cDNA in a suitable expression system using established recombinant DNA methods. Production of the cardiac adenylyl cyclase is achieved by incorporation of the cardiac adenylyl cyclase cDNA into any suitable expression vector and subsequent transformation of an appropriate host cell with the vector; alternatively, the transformation of the host cell is achieved directly by naked DNA without the use of a vector. Production of the cardiac adenylyl cyclase by either eukaryotic cells or prokaryotic cells is contemplated by the present invention. Examples of suitable eukaryotic cells include mammalian cells, plant cells, yeast cells and insect cells. Similarly, suitable prokaryotic hosts, in addition to E. coli, include Bacillus subtilis.

Other suitable expression vectors are employed and are selected based upon the choice of host cell. For example, numerous vectors suitable for use in transforming bacterial cells are well known. For example, plasmids and bacteriophages, such as λ phage, are the most commonly used vectors for bacterial hosts, and for E. coli in particular. In both mammalian and insect cells, viral vectors are frequently used to obtain expression of exogenous DNA. In particular, mammalian cells are commonly transformed with SV40 or polyoma virus; and insect cells in culture are transformed with baculovirus expression vectors. Yeast vector systems include yeast centromere plasmids, yeast episomal plasmids and yeast integrating plasmids.

It will also be understood that the practice of the invention is not limited to the use of the exact sequence of the cardiac adenylyl cyclase cDNAs as defined in Figure 2 (Sequence ID No. 1). Modifications to the sequence, such as deletions, insertions, or substitutions in the sequence which produce silent changes in the resulting protein molecule are also contemplated. For example, alterations in the cDNA sequence which result in the production of a chemically equivalent amino acid at a given site are contemplated; thus, a codon for the amino acid alanine, a hydrophobic amino acid, can readily be substituted by a codon encoding another hydrophobic residue, such as glycine, or may be substituted with a more hydrophobic residue such as valine, leucine, or isoleucine. Similarly, changes which result in substitution of one negatively charged residue for another, such as aspartic acid for glutamic acid, or one positively charged residue for another, such as lysine for arginine, are expected to produce a biologically equivalent product.

Nucleotide changes which result in alteration of the N-terminal and C-terminal portions of the protein molecule frequently do not alter protein activity, as these regions are usually not involved in biological activity. It may also be desirable to eliminate one or more of the cysteines present in the sequence, as the presence of cysteines may result in the undesirable formation of multimers when the protein is produced recombinantly, thereby complicating the purification and crystallization processes.

Each of the proposed modifications is well within the routine skill in the art, as is determination or retention of biological activity of the encoded products. Therefore, where the phrase "cardiac adenylyl cyclase cDNA sequence" or "cardiac adenylyl cyclase gene" is used in either the specification or the claims, it will be understood to encompass all such modifications and variations which result in the production of a biologically equivalent cardiac adenylyl cyclase protein. It is also understood to include the corresponding sequence in other mammalian species. In particular, the invention contemplates those DNA sequences which are sufficiently duplicative of the sequence of Figure 2 or the adenylyl cyclase nucleic acid in clone number 341 (ATCC No. ) so as to permit hybridization therewith under standard high stringency southern hybridization conditions, such a those described in Maniatis et al. (16).

In an example of such expression, twenty µg of the purified plasmid pcDNA 113-72 are transfected into the monkey kidney CMT cells using a modified method of Golub et al. (17). Briefly, the cells are grown to 80% confluence in Dulbecco's modification of Eagle's Medium, 10% fetal calf serum, 2 mM glutamine, 4.5 mg/ml glucose, 10 µg/ml streptomycin sulfate and 60 µg/ml penicillin K. After washing with PBS twice, 0.5 ml of trypsin solution is added. The cells are incubated for 10 minutes, and 20 µg of purified plasmid resuspended in 4 ml of DMEM containing 400 µg/ml DEAE dextran and 0.1 mM chloroquine is added. The cells are incubated for four hours followed by 10% DMSO shock for two minutes. After washing with PBS twice, the induction media, which contains 10% fetal calf serum (FCS), 2 mM glutamine, 4.5 g/ml glucose, 2 mM penicillin and streptomycin, and 1 µM CdCl₂, 0.1 µM ZnCl₂ in DMEM, is added. The plate is incubated at 37^{o}C for 72 hours before harvesting.

This adenylyl cyclase protein, composed of 1184 amino acids, is analyzed for secondary structure by the method of Kyte-Doolittle (11) (Figure 3). The software, MacVector 3.5 (IBI, New Haven, CT), is used to obtain a hydropathy plot and thereby identify the membrane related structure of this cardiac adenylyl cyclase. The method of Kyte and Doolittle is used with a window size of 7.

As shown in Figure 3, twelve peaks are numbered. These peaks represent transmembrane spanning regions. These results suggest that this cardiac adenylyl cyclase possesses a structure of twelve transmembrane spanning regions, as well as a large cytoplasmic loop located in the middle and at the end. In the transmembrane positions, the fifth extracellular loop is the largest (between the ninth and tenth transmembrane spans).

164 of the N-terminal tail is located within the cytoplasm, followed by a 6-transmembrane spanning region of 154 amino acids (amino acid position 165-318). Then 366 amino acids of the cytoplasmic domains (319-684) precede the second 6-transmembrane spanning domain of 243 amino acids (685-927), followed by another cytoplasmic domain of 256 amino acids (928-1184). Thus it makes a duplicated form of 6-transmembrane spanning region and large hydrophobic cytoplasmic domain.

As shown in Figure 4, a protein dot matrix comparison among type I, type III and the cardiac adenylyl cyclase, the two large hydrophobic cytoplasmic loops show homology of ∼50% with each other. However, the homology between the two transmembrane spanning portions is very low (less than 30%).

The membrane associated secondary structure of the protein (based on the results of Figure 3) is well conserved among different types of mammalian adenylyl cyclases (types I, II, III, and cardiac types). All of them possess two large cytoplasmic loops, interrupted by the presence of 6-transmembrane spanning region. The homology among the different types of adenylyl cyclase is only conserved in the cytoplasmic portions, even though the other portions are structurally similar. Furthermore, in the same adenylyl cyclase protein, the homology between the two cytoplasmic portions is also maintained. This suggests the cytoplasmic portion is a result of gene duplication.

It has been suggested that the level of activity of the adenylyl cyclases in the heart correlates with the development of heart failure. There is a significant decrease in the cyclase activity in the failed heart compared with that in the non-failed heart (10,18,19,20). These papers suggest that there is a distal regulation in the signal transduction pathway, i.e., the regulation at the level of cyclase. In fact, the decreased activity of adenylyl cyclase in the heart may be the major factor in the development of heart failure.

A pacing-induced canine model of heart failure in the left ventricle (LV) is used to identify the following: 1) an uncoupling of the beta-adrenergic receptor from G_{S}, i.e., loss of high-affinity binding 44 ± 1 to 30 ± 2 fmol/mg), 2) no change in the activity of G_{S} itself, and 3) a progressive decline of adenylyl cyclase catalytic activity is associated with a change in the steady state mRNA levels encoding from the cloned adenylyl cyclase isoform which is predominantly expressed in the heart. A decrease in the adenylyl cyclase mRNA levels paralleled both the progressive decline in left ventricle function, e.g., heart rate (HR)beat/min., left ventricle end-diastolic pressure (LVEDP, mmHG), that occurred from 1-4 weeks of pacing as well as the loss in sarcolemma adenylyl cyclase catalytic activity (pmol/min.mg.protein).

| | HR | LVEDP | AC Activity | ACmRNA | Number |
|---|---|---|---|---|---|
| Control | 89±4 | 4.3±0.3 | 97±6 | 10±0.08 | 4 |
| 1 week | 117±2 | 11±2 | 65±4 | 0.74±0.07 | 3 |
| 4 week | 136±13 | 33±4 | 45±3 | 0.41±0.03 | 3 |

This data shows that a decrease in the content of the adenylyl cyclase catalyst itself contributes to impaired cyclic AMP production in heart failure.

The novel cardiac adenylyl cyclase of this invention is used to screen for compounds which stimulate the activity of that cyclase.

The biochemical property of this cardiac adenylyl cyclase is examined in a transient expression system using CMT cells (a derivative of COS cells). CMT cells contain T-antigen driven by a methalothionein promoter in the genome. Thus by induction with heavy metal ion in the medium, CMT cells could produce more T-antigen than COS cells. A 4.3 kb fragment of the adenylyl cyclase cDNA (#113-72) containing the whole coding sequence is inserted into the pcDNA1 plasmid described above (Invitrogen).

The adenylyl cyclase activity of a cell transfected with the expression vector pcDNA1 carrying cardiac adenylyl cyclase cDNA is assayed as follows. The transfected CMT cells are washed twice with PBS and scraped in three ml of cold buffer containing 50 mM Tris (pH8.0), 1 mM EDTA, 10 µM PMSF (phenylmethylsulfonylfluoride), 100 U leupeptin, and 50 U egg white trypsin inhibitor (ETI) on ice. The membrane is homogenated in Polytron™ (setting 6 for 10 seconds) and is centrifuged at 800 x g for 10 minutes at 4^{o}C. The supernatant is further centrifuged at 100 x g for 40 minutes at 4^{o}C. The resultant pellet is resuspended in 50 mM Tris (pH 8.0), 1 mM EDTA, 1 µM PMSF, 50 U leupeptin, and 50 U ETI, to a concentration of 5 µg/µl. This crude membrane solution is used for the adenylyl cyclase asssay.

The adenylyl cyclase assay is performed by the method of Salomon (21). Briefly, the crude membranes from CMT cells are resuspended in a solution containing 1 mM creatine phosphate, 8 µg/ml creatine phophokinase, 4 mM HEPES (pH 8.0), 2 mM MgCl₂, 0.1 mM c-AMP, 0.1 mM ATP, and ³²P-ATP (0.2-5 µCi/assay tube). The reaction mixture is incubated at 37^{o}C for 30 minutes and the reaction is stopped by the addition of 100µl 2% sodium dauryl sulfate. To monitor the recovery from the column, ³H-labelled c-AMP is used. Cyclic-AMP is separated from ATP by passing through Dowex and alumina columns, and the radioactivity is counted by scintillation counter. The protein concentration of the membranes used are measured by Bradford's method (22), with bovine serum albumin as a standard.

The membrane from untransfected CMT cells is used as a control. The results of the adenylyl cyclase activity assay are shown in Table 1:

**Table 1**

| | Basal | NaF | GTPγS | Forskolin |
|---|---|---|---|---|
| Control | 2.5±0.5 | 15±3.1 | 30± 4.5 | 52± 7.2 |
| Transfected | 11.0±1.6 | 41±4.7 | 84±12.5 | 321±28.0 |

The adenylyl cyclase expressed by this cDNA is well stimulated by 10 mM sodium fluoride, 100 µM GTPγS and 100 µM forskolin. It shows 2.7, 2.8, and 6.2 fold more stimulation than the control. Values are shown with ± standard error.

The adenylyl cyclase activity is also stimulated more than 10-fold over the control in the presence of 5 mM manganese. An increased basal activity of adenylyl cyclase in the transfected cells is also observed. This suggests that this cyclase possesses high basal activity, allowing high accumulation of cyclic AMP in the heart. This is consistent with the high basal cyclase activity seen in cardiac tissue.

In order to clarify the tissue distribution of the cardiac adenylyl cyclase (A form), Northern blotting is performed using mRNA from various tissues. Messenger RNA is purified using guanidium sodium (23) and oligo-dT columns from various canine tissues (heart, brain, testis, skeletal muscle, kidney and lung). Five µg of mRNA are used for each assay (per lane of blot).

The blot is prehybridized in a solution containing 50% formamide, 5 x SSC, 5 x Denhardt's, 25mM NaPO₄ (pH6.5), 0.25 mg/ml calf thymus DNA, and 0.1% SDS at 42^{o}C for two hours before the addition of a probe. A 0.9 kb EcoRI-HincII restriction fragment from the adenylyl cyclase cDNA is used as a probe. The probe is made by a multiprimer random labelling method with ³²P-dCTP. Hybridization is performed at 42^{o}C for 18 hours followed by washing under increasingly stringent conditions. The blot is then autoradiographed.

The results of the Northern blot analysis, as depicted in Figure 5, show that the message is most abundant in the heart, as well as in the brain, but rarely expressed in other tissues, such as testis, skeletal muscle, kidney and lung. In the heart and brain there are two different sizes of message, whose sizes are 5 and 7 kb (Figure 5). The 5 kb message is more abundant. The ratio of the two messages is estimated by a radiodensitometer to be 3:2. The two messages are also observed when a fragment from a 3' untranslated portion of the cDNA (ApaI-XhoI, a 0.3 kb fragment) is used as a probe.

The whole protein is encoded by 3552 bases. This suggests that the mRNA contains at least 2.0 to 4.0 kb of untranslated sequence.

In a separate series of experiments, a cDNA library is prepared according to standard procedures in a λgt10 vector using poly(A)⁺ RNA prepared from canine ventricular tissue. In the primary library screening, 2.0 x 10⁶ independent clones are screened with EcoRI-SphI fragment from Type V adenylyl cyclase cDNA as a probe. The hybridization is carried out in a solution of 30% formamide, 5 x SSC, 5 x Denhardt's, 25 mM NaPO₄ (pH 6.5), 0.25 mg/ml calf thymus DNA and 0.1% SDS at 42^{o}C. Hybridization is carried out in the same buffer at 42^{o}C for 14-20 hours with cDNA fragments labelled with ³²P, followed by washing under increasingly stringent conditions. In later screens, a hybridization solution with 50% formamide instead of 30% is used. All DNA sequencing (using either universal or synthetic oligonucleotide primers) is carried out bidirectionally at least twice using either Sequenase or Taq polymerase. For certain GC-rich sequences, such as the 5' untranslated region, the reaction is carried out both with Sequenase and Taq polymerase with or without 7-deaza-dGTP. Electrophoresis is carried out in a polyacrylamide gel containing 8 M urea and 20% formamide to eradicate problems with band compression. For genomic DNA cloning, 3 x 10⁶ recombinant clones from EMBL3 canine genomic DNA library are screened with a probe from ATCC No. 68968 (type V adenylyl cyclase). Screening and sequencing are performed similarly as described above.

In a second series of experiments, a 2.1 kb, EcoRI-StuI fragment, designated as #113-α, was constructed by ligating the EcoRI-SphI (nucleotide 1 to 954, from #72 in puc18), SphI-EcoRI (nucleotide 954 to 1604, from #7), and EcoRI-SspI (nucleotide 1604 to 2137, from #6L) fragments. An EcoRI-StuI fragment designated as #113-β is constructed by digesting #113 with EcoRI and SspI (nucleotide 1639 to 4393 from Type V adenylyl cyclase). Each fragment is subcloned into the unique polylinker site of the plasmid pcDNA I-amp, a mammalian expression vector, and designated pcDNA113-α and -β. Twenty µg of the purified plasmid are transfected into CMT cells by a modification of the method of Golub et al. Briefly, the cells are grown to 80% confluence. After washing with phosphate buffered saline (PBS) twice, 0.5 ml of trypsin solution is added. The cells are incubated for 10 minutes, and 20 µg of purified plasmid resuspended in 4 ml of DMEM containing 400 µg DEAE dextran and 0.1 mM chloroquine are added. The cells are incubated for 4 hours followed by DMSO shock for 2 minutes. After washing with PBS twice, the induction medium which contains 1 µM CdCl₂ and 0.1 µM ZnCl₂, was added and the cells are incubated at 37^{o}C for 72 hours before harvesting. Control cells were mock-transfected and induced in the same way.

Two primers are obtained from the novel sequence from #6L. Poly(A)⁺ mRNA is isolated from total RNA by binding with oligo(dT) cellulose. Two µg of poly(A)+mRNA were used or reverse transcriptase using cDNA Cycle Kit (Invitrogen, CA). The reaction is incubated at 42^{o}C for 1 hour, heated to 95^{o}C and then quickly chilled on ice. One-tenth of the PCR product is electrophoresed in 2% agarose gel, and transferred to nitro cellulose and hybridized with a probe. Sixty mer oligonucleotide primer is used as a probe, whose sequence is taken from the 3' end of the Type V-α cDNA. Hybridization is performed similarly as described above except that 30% formamide solution is used instead of 50% formamide solution.

The transfected CMT cells are washed twice with PBS and then collected into 1 ml of cold buffer containing 50 mM Tris HCl (pH 8.0), 2 mM EGTA, 10 µM PMSF (phenylmethylsulfonylflouride), 100 U leupeptin, and 50 U ETI (egg white trypsin inhibitor). The cells are homogenized with a Polytron (setting 6 for 10 seconds) and centrifuged at 800 x g for 10 minutes at 4^{o}C. The supernatant is further centrifuged at 100,000 x g for 40 minutes at 4^{o}C. The resultant pellet is resuspended in 50 mM Tris (pH 8.0), 1 mM EDTA, 1 µM PMSF, 50 U leupeptin and 50 U ETI, to a concentration of 5 µg/µl. This crude membrane preparation is used in the adenylyl cyclase assay.

Adenylyl cyclase activity is measured by the method of salomon. Briefly, the washed membranes from CMT cells (15-30 µg per assay tube) are resuspended in 100 µl of solution containing 1 mM creatine phosphate, 8 U/ml creatine phosphokinase, 4 mM Hepes (pH 8.0), 2 mM MgCl₂, 0.1 mM cyclic AMP, 0.1 mM ATP, and ³²P-ATP (0.2-5 µCi/assay tube). The reaction mixture is incubated at 30^{o}C for 30 minutes and the reaction is stopped by the addition of 100 µl 2% SDS. To monitor the recovery from the columns, ³H-labelled cyclic AMP is used. Cyclic AMP is separated from ATP by passing through Dowex and alumina columns, and the radioactivity is measured by liquid scintillation counting. Protein concentration is measured by the method of Bradford using bovine serum albumin as a standard.

Using an EcoRI-SphI probe, 12 primary positives are obtained. All the clones are subcloned into puc18 vector and sequenced. Ten out of 12 clones are identical to Type V adenylyl cyclase clone. Two clones, #6L and #2, are identical in the 5' half of the sequence, however, totally diverged from Type V clone at its 3'-end. It had -570 bp of unique sequence followed by a polyadenylation signal (Figure 1A). The 5' end of the clone 6L extended to the position 150 bp downstream from the translation initiation site. Clone #2 was similar to clone #6L, except that it extended less upstream than clone #6L. Several other clones are identified extending to different length both upstream and downstream, all of which possessed the novel sequence at the same portion and utilized the same polyadenylation signal.

A canine genomic library is screened using this 200 bp of novel sequence as a probe. Two million independent colonies are screened. Five positives are obtained, one clone, #123, is further screened. The 10 kb insert from the clone #123 is restriction mapped and the fragments are subcloned into puc18 plasmid. A 4 kb EcoRI-EcoRI fragment from the 10 kb insert is found to hybridize to the unique probe. This insert is sequenced partially and the results are shown in Figure 1B. The unique sequence obtained from the clone 6L and 2 is identical to that from the gene fragment (#123); it is identical to that of the intron followng the exon-intron border portion. Indeed, the consensus sequence of acceptor donor site is maintained in this clone (...AAGCGG**GTCC**...).

This suggests that the transcription of the mRNA was terminated at the middle of the molecule in this novel cDNA because of the presence of an alternative polyadenylation site in the intron ∼570 bp downstream from the regular exon-intron junction site. Thus, the matured mRNA of this truncated form of adenylyl cyclase contains a part of the intron sequence followed by polyadenylation. The consensus sequence of this polyadenylation site is not in a common manner, thus, the amount of the message utilizing this polyadenylation site would be minor. It makes a protein composed from 596 amino acids, 25 amino acids out of which are unique to this clone.

An 2.8 kb EcoRI fragment from the clone 6L is subcloned into pcDNA, termed as pcDNA 113-α, using its unique restriction sites. A 2.4 kb EcoRI-SspI fragment from clone #113 is cloned into pcDNA, termed as pcDNA 113-β. The adenylyl cyclase activities of three clones are compared; #113-72, which encodes a conventional full molecule, #113-α, which encodes an initial half of the molecule, and #113-β, which encodes a latter half of the molecule. When expressed alone, neither #113-α nor #113-β had an increased activity over the mock-transfected control. However, when co-transfected together, the activity was enhanced significantly. More than 70% of the activity of the full-molecule (#113-72) is recovered. This enhancement was seen both in the basal and stimulated with GTPγS, NaF and forskolin.

The above data suggest that Type V-α alone does not have any adenylyl cyclase activity, as yet when co-expressed with its counter partner, Type V-β, it could possess the activity. This is inconsistent with the observation of other investigators. The finding that such a half molecule does exist for Type V adenylyl cyclase suggests that the basic subunit of adenylyl cyclase could be a 6-transmembrane motif like as other members of the family.

Canine cardiac adenylyl cyclase polypeptides are synthesized by the solid-phase Merrield method on an Applied Protein Technologies synthesizer (Cambridge, MA) and are purified by high-performance liquid chromatography to >99%. The purity is confirmed by mass spectrophy. Peptides synthesized have the following sequences. #1, E³⁴⁶-E³⁶⁵; #2, K⁴²⁵-C⁴⁴⁴;, #3, K⁶²⁶-A⁶⁴⁵; #4, C⁸⁰¹-S⁸²⁵; #5, A⁸⁸⁶-T⁹⁰⁵;, #A, M⁵⁸¹-G⁵⁹⁰ ; #B, Y¹¹⁷⁵-S¹¹⁸⁴; #C, Q¹¹⁴²-N¹¹⁵⁴;, #6, C⁵⁰⁰-Y⁵¹⁹.

Table 2 shows the effects of various peptides on GTPλS-stimulated type V adenylyl cyclase activity in CMT cells. Peptides used differ in size from 4 to 25 amino acids. The sequence of the peptide is taken from the intracellular (#1, #2, #3, #6, #A, #B and #C), extracellular #4 and #5) or transmembrane (#D) domains. At the concentration of 1 x 10⁻⁴ M, #2 and #6 peptide show a significant inhibition on the catalytic activity, while rest of the peptides show no significant effects. It is of interest that more than 90% of the catalytic activity is suppressed in the presence of the peptide, #2 or #6.

The unique character of these peptides also is utilized in drug screening procedures. First, the peptides themselves are specific inhibitors of adenylyl cyclase when administered to a subject, locally or systematically. Secondly, putative antibodies raised against each peptide possess the same inhibitory effect as described above. Thirdly, compounds, which would compete with the reaction site with the peptide to the adenylyl cyclase molecule, are screened by utilizing this peptide inhibition assay. Such compounds have biological effect on the catalytic activity of the molecule.

**TABLE 2**

| **Percent Inhibition of the Adenylyl Cyclase Activity in the Presence of Peptides** | |
|---|---|
| | % Inhibition |
| Control | 0 |
| #1 | <5 |
| #2 | >90 |
| #3 | <5 |
| #4 | <5 |
| #5 | <5 |
| #6 | >90 |
| #A | <5 |
| #B | <5 |
| #C | <5 |

### Bibliography

1. Salter, R.S., et al., J. Biol. Chem., 256, 9830-9833 (1981).
2. Pfeuffer E., et al., EMBO J., 4, 3675-3679 (1985).
3. Mollner, S., et al., Eur. J. Biochem., 195, 281-286 (1991).
4. Livingstone, M.S., et al., Cell, 37, 205-215 (1984)).
5. Krupinski, J., et al., Science, 244, 1558-1564 (1989)
6. Tang, W-T, et al., J. Biol. Chem., 266, 8595-8603 (1991).
7. Bakalyar, H.A., and Reed, R.R., Science, 250, 1403-1406 (1990).
8. Pfeuffer, E., et al., Proc. Natl. Acad. Sci. USA., 82, 3086-3090 (1985).
9. Mollner, S., and Pfeuffer, T., Eur. J. Biochem., 171, 265-271 (1988).
10. Chen, L., et al., J. Clin. Invest., 87, 293-298 (1991).
11. Kyte, J., and Doolittle, R.F., J. Mol. Biol., 157, 105-132 (1982).
12. Watson, C.J. and Jackson, J.F., in DNA Cloning: A Practical Approach, Glover, D.M., ed., vol. 1, pp.79-88 (1985).
13. Tabor, S., and Richardson, C.C., Proc. Natl. Acad. Sci. USA, 84, 4767-4771 (1987).
14. Innis, M.A., et al., Proc. Natl. Acad. Sci. USA, 85, 9436-9440 (1988).
15. Kozak, M., J. Cell. Biol., 108, 229-241 (1989).
16. Maniatis et al., Molecular Cloning: A Laboratory Manual, Cold spring Harbor Laboratory, (1982).
17. Goolub, E.I., et al., Nucleic Acid Research, 17, 4902 (1989).
18. Robberecht, P., et al., Biochem. Pharmacol., 30, 385-387 (1981).
19. Chatelain, P., et al., Eur. J. Pharmacol., 72, 17-25 (1981).
20. Palmer, G.C., and Greenberg, S., Pharmacology, 19, 156-162 (1979).
21. Salomon, Y., Adv. Cyclic Nucleotide Res., 10, 35-55 (1979).
22. Bradford, M., Anal. Biochem., 72, 248 (1976).
23. Chomczynski, P., and Sacchi, N., Anal. Biochem., 162, 156-159 (1987).
24. Katsoka, T. et al., Cell, 43:493-505 (1985).
25. Chinkers, M. et al., Nature, 338:78-83 (1989)
26. Singh, S. et al., Nature, 384:708-712 (1988).
27. Lynch, T. et al., Biochem. Biophys. Res. Comm., 68:6116-6125 (1976).

## Claims

1. An isolated nucleic acid molecule encoding a cardiac adenylyl cyclase type V.

2. The isolated nucleic acid molecule of Claim 1, wherein the nucleic acid molecule is a nucleic acid selected from the group consisting of DNA, cDNA, or RNA.

3. The isolated nucleic acid molecule of Claim 1, wherein the nucleic acid molecule is a mammalian nucleic acid molecule.

4. An isolated polypeptide encoded by the nucleic acid molecule of Claim 1.

5. An expression vector which comprises the nucleic acid molecule of Claim 1.

6. A host cell stably transformed comprising the expression vector of Claim 5.

7. An antibody capable of forming a complex with the polypeptide of Claim 4.

8. A method for producing a cardiac adenylyl cyclase type V polypeptide which comprises growing the host cell of Claim 6 under conditions favoring the production of the polypeptide and recovering the polypeptide so produced.

9. A method for determining cardiac function in a subject which comprises:
a) isolating a suitable RNA sample from the subject;
b) determining the amount of RNA in the sample by hybridizing the cDNA of Claim 4 to the RNA in the sample.

10. A method of modifying cardiac function in a patient which comprises administering to the patient the pharmaceutical composition of Claim 10.
